## Europäisches Patentamt
## European Patent Office
## Office européen des brevets

(19)

(11) Veröffentlichungsnummer: **0 192 129**
**B1**

(12)

# EUROPÄISCHE PATENTSCHRIFT

(45) Veröffentlichungstag der Patentschrift: **26.09.90**

(51) Int. Cl.⁵: **G 01 N 33/48,** G 01 N 1/28

(21) Anmeldenummer: **86101507.1**

(22) Anmeldetag: **05.02.86**

(54) **Verfahren zur Herstellung von Reagenzlösungen.**

(30) Priorität: **19.02.85 DE 3505631**

(43) Veröffentlichungstag der Anmeldung:
**27.08.86 Patentblatt 86/35**

(45) Bekanntmachung des Hinweises auf die
Patenterteilung:
**26.09.90 Patentblatt 90/39**

(84) Benannte Vertragsstaaten:
**BE CH DE FR GB IT LI NL**

(56) Entgegenhaltungen:
**DE-A-3 137 014**

(73) Patentinhaber: **MERCK PATENT GESELLSCHAFT MIT BESCHRÄNKTER HAFTUNG**
**Frankfurter Strasse 250 Postfach 4119**
**D-6100 Darmstadt (DE)**

(72) Erfinder: **Limbach, Berthold, Dr.**
**Otto-Hahn-Strasse 8**
**D-6104 Seeheim Jugenheim (DE)**
Erfinder: **Helger, Roland, Dr.**
**Ludwigshöhstrasse 85**
**D-6100 Darmstadt (DE)**

Courier Press, Leamington Spa, England.

# EP 0 192 129 B1

## Beschreibung

Die Erfindung betrifft ein Verfahren zur Herstellung von Reagenzlösungen, die eine Mehrzahl von Substanzen enthalten.

Reagenzlösungen, insbesondere solche für klinischchemische Analysen, enthalten eine Vielzahl verschiedener Substanzen, wie die für die eigentliche Analyse erforderlichen Reaktionspartner (Enzyme, Coenzyme, Substrate, Indikatoren) sowie Hilfsstoffe (Puffer, Detergenzien) und Stabilisatoren. Zur Durchführung der Analyse müssen diese Substanzen in einem festgelegten Schema und in genauer Dosierung zusammengegeben werden, da nur so die hohen Anforderungen an Präzision und Richtigkeit erfüllt werden können. Andererseits erfordert die hohe Anzahl täglich durchzuführender Analysen eine für den Anwender möglichst einfache und schnelle Testvorbereitung unabhängig davon, ob die herzustellende Reaktionslösung für Bestimmungen im einfachen Handtest oder für ein Analysensystem mit großem Probendurchsatz benötigt wird. In der Regel wird deshalb versucht, für einen bestimmten Test möglichst viele Substanzen in einem Lösungs- oder Feststoffgemisch zusammenzufassen, die dann durch Vermischen mit Wasser die einsatzbereiten Reaktionslösungen ergeben. Da die benötigten Substanzen zum Teil nur in sehr geringen Mengen benötigt werden, die sich als Feststoffe nicht ohne weiteres mit der erforderlichen Reproduzierbarkeit mischen lassen, erfolgt die Mischung üblicherweise in Form von Lösungen, die aus Stabilitätsgründen anschließend lyophilisiert werden. Dieser Prozeß ist sehr zeit- und energieaufwendig. Darüberhinaus führt das Zusammenbringen mehrerer Substanzen in einer Mischung mitunter zu Unverträglichkeiten, vor allem deshalb, weil die einzelnen Substanzen nicht bei den jeweils optimalen Bedingungen (pH-Wert, Stabilisatoren) bearbeitet und anschließend gelagert werden können. So ist letztlich in manchen Fällen ein einziges Gesamtgemisch nicht realisierbar; durch Herstellung mehrerer Gemische werden aber Aufwand und Kosten erhöht. Dies gilt ebenso für das getrennte Einfrieren und Trocknen von Substanzen in einem Gefäß bei sogenannten "Schichtenlyophilisaten".

Es ist bekannt, daß porige, preßfähige Materialien die Fähigkeit besitzen, Stoffe, insbesondere Flüssigkeiten, in sich aufzusaugen und zu halten, um sie bei Anwendung von äußeren Druck reversibel abzugeben. Auf diese Weise ist eine leichte und reproduzierbare Handhabung von Flüssigkeiten möglich. In der DE—A—2559574 ist z.B. eine Vorrichtung zum Aufbringen von Pufferlösung auf einen Analysenfilm beschrieben. Diese Vorrichtung enthält ein Paar liegender Walzen mit einem Oberflächenbelag aus einem schwammartigen Material, von denen wenigstens eine mit Pufferlösung beschickt ist. Der Analysenfilm wird durch den Walzenspalt mit Pufferlösung benetzt und bei seinem Austritt aus dem Walzenspalt von überflüssiger Pufferlösung durch Aufsaugen in dem schwammartigen Material wieder befreit.

In der DE—A—3137014 ist ein Testsystem beschrieben, das mindestens eine mit Substanzen imprägnierte, preßfähige Matrix und eine Vorrichtung enthält, durch die die Matrix nach Aufnahme der zu untersuchenden Flüssigkeit unter Abgabe des Reaktionsproduktes komprimiert wird. Das Imprägnieren der Matrix erfolgt dabei durch Tränken mit einer Lösung der jeweils benötigten Substanzen und anschließende Lyophilisation; unverträgliche Substanzen können dabei getrennt voneinander aufgegeben werden. Die preßfähige Matrix ist dabei sowohl Trägermaterial für die Testsubstanzen als auch gleichzeitig Reaktionsort für die eigentliche Tetreaktion. Deshalb ist es nicht erforderlich, daß alle Substanzen reproduzierbar und nahezu vollständig aus der Matrix eluiert werden, da zu diesem Zeitpunkt die eigentliche Testreaktion bereits abgeschlossen ist. Es ist u.U. sogar wünschenswert, daß Substanzen einer ersten Teilreaktion nicht mit in die nächste Teilreaktion übernommen werden. Hingegen müssen bei der Herstellung von Reaktionslösungen alle Substanzen reproduzierbar und möglichst vollständig von dem Trägermaterial eluiert werden, um eine genügend genaue Bestimmung zu ermöglichen.

Die bekannten Systeme beschreiben Manipulationen von zu untersuchenden Flüssigkeiten, ohne daß die zur Herstellung von Reagenzlösungen erforderlichen Verfahren der reproduzierbaren Auflösung und Verteilung von zum Teil untereinander nicht verträglichen, aus Stabilitätsgründen als Feststoffe vorliegenden Substanzen erwähnt werden.

Der Erfindung liegt die Aufgabe zugrunde, ein Verfahren zur Herstellung von Reagenzlösungen zur Verfügung zu stellen, wobei eine Vielzahl von Substanzen in trockener Form auf einem festen Trägermaterial vorliegen.

Gegenstand der Erfindung ist ein Verfahren zur Herstellung von mehrere Substanzen enthaltenden Reagenzlösungen zur Durchführung von Analysen, wobei die Substanzen auf mindestens ein Element eines festen porigen und preßfähigen Trägermaterials aufgebracht sind, das dadurch gekennzeichnet ist, daß die Herstellung der Reagenz lösung durch Einsaugen eines Reagenzlösungsmittels in das Trägermaterial und anschließendes Ausdrücken erfolgt. Als poriges und preßfähiges Trägermaterial wird vorzugsweise ein offenzelliger Polyurethanschaumstoff verwendet.

Überraschenderweise wurde gefunden, daß feste, porige, preßfähige Materialien als Träger zur Herstellung von Reagenzlosungen dienen können. Besonders überraschend ist, daß selbst bei Verwendung von im Vergleich zu anderen Trägern größervolumigen Materialien eine schnellere und besser reproduzierbare Ausspülung von Reagenzien möglich ist. Erreicht wird dies durch mehrfaches Ausdrücken und Entspannen der imprägnierten Matrixelemente in dem Reagenzlösungsmittel. Das in die Matrix aufgesaugte Reagenzlösungmittel verbleibt dabei nur kurzzeitig in der Matrix, um die aufgebrachten Substanzen abzulösen und in die Gesamtlösung zu überführen, wo dann die eigentliche Rekonstitution der Reagenzien stattfindet. Nach drei- bis viermaliger Wiederholung des Preßvorganges wird so eine

2

reproduzierbare Ausspülung der aufgebrachten Substanzen erreicht, auch wenn die jeweilige Verweildauer des Reagenzlösungsmittels in der Matrix deutlich kürzer ist als die zur vollständigen auflösung der Substanz erforderliche Zeit. Zugleich wird durch den Preßvorgang auch eine Durchmischung der Gesamtlösung erzielt. Die nach dem erfindungsgemäßen Verfahren hergestellte Reagenzlösung kann insgesamt oder als aliquoter Teil zur Durchführung von Analysen eingesetzt werden.

Als feste, porige, preßfähige Materialien können alle Stoffe verwendet werden, die in der Lage sind, eine wäßrige Lösung reversibel in sich aufzunehmen und bei der Anwendung von äußerem Druck möglichst vollständig wieder abzugeben. Der Ausdruck reversibel bedeutet dabei, daß dieser Vorgang beliebig wiederholbar ist. Die Matrix selbst wird bei diesem Vorgang gleichfalls reversibel auf einen Bruchteil ihres Volumens zusammengepreßt, ohne daß sie dabei zerfällt oder eine Form annimmt, die ein leichtes Entfernen aus dem Reagenzlösungsmittel nicht mehr erlauben würde.

Geeignete Materialien sind natürliche und synthetische schwamm- und schaumstoffartige, poröse Stoffe, zum Beispiel offenzellige Formkörper aus saugfähigen Materialien, vorzugsweise offenzellige Polyurethanschäume, wie Polyetherschäume, Polyesterschäume oder Polyether-Verbundschäume.

Die Stauchhärte dieser Materialien sollte im Bereich von etwa 2 bis 10 kPa liegen, wobei solche mit einer Stauchhärte unter 2 kPa schlechte mechanische Eigenschaften zeigen, solche über 10 kPa erfordern einen zu großen Druck bei der Flüssigkeitsabgabe.

Die Matrix kann aus einem Stück oder aus mehreren Lagen oder Segmenten bestehen. Die Anzahl richtet sich nach de Anzahl der getrennt voneinander aufzugebenden Substanzen. Selbstverständlich ist es auch möglich, mehrere Lagen eines mit einer Substanz getränkten Materials einzusetzen; das ist z.B. bei Testverfahren erforderlich, bei denen die gleiche Substanz in verschiedenen Konzentrationen eingesetzt wird.

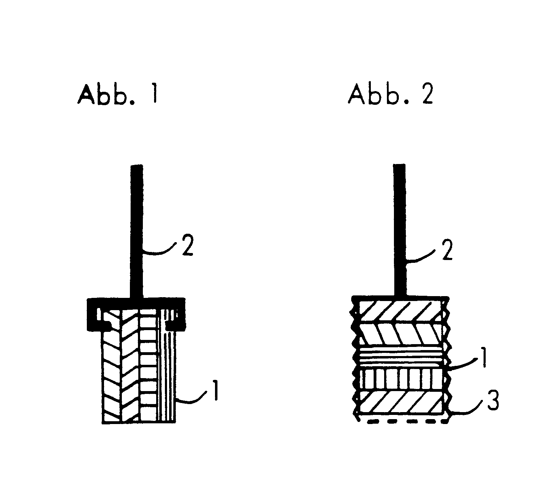

Die einzelnen Teile können durch Verkleben oder Vernetzen miteinander verbunden werden oder aber durch eine entsprechende Vorrichtung zusammengehalten werden. Dabei können die einzelnen Matrixlagen in horizontaler oder vertikaler Anordnung geschichtet sein. Die Vorrichtung ist vorzugsweise so konstruiert, daß die Matrixlagen bzw. -segmente in eine das Reagenzlösungsmittel enhaltendes Vorratsgefäß eingeführt werden können und daß ein mehrfaches, möglichst vollständiges Zusammendrücken und Entspannen des Trägermaterials und daran anschließend sein Entfernen aus dem Vorratsgefäß möglich ist. In den Abbildungen 1 und 2 sind zwei mögliche einfache Konstruktionen dargestellt. Abb. 1 zeigt einen festen Halter (2), an dessen Ende die einzelnen Matrixlagen (1) eingeklemmt sind. Durch Niederdrücken des Halters auf dem Boden des Vorratsgefäßes wird das Trägermaterial zusammengepreßt, bei nachlassendem Druck entspannt es wieder. In Abb. 2 ist eine Kammer aus flexiblem, an seinem Boden durchlöchertem Kunststoff (3) dargestellt, in die die einzelnen Matrixteile (1) horizontal eingelegt sind. Am oberen Ende der Kammer ist der Halter (2) angebracht. Beim Zusammenpressen im Vorratsgefäß erfolgt ein Zusammendrücken der Matrix, anschließend wird beim Entspannen ein Teil der im Vorratsgefäß befindlichen Lösung durch die Löcher in dem flexiblen Kunststoff in das Trägermaterial gesaugt und beim erneuten Zusammendrücken wieder in die Gesamtlösung abgegeben. Die Eigenschaften der Matrix bei Anwendung in dem erfindungsgemäßen Verfahren erlauben dabei eine mehrfache Wiederholung dieses Prozesses ohne weitere Hilfsmittel. Möglich ist auch die Verwendung von Spritzen-ähnlichen Hilfsmitteln wie Enwegspritzen, in die das Reagenzlösungsmittel eingesaugt werden kann. Es handelt sich dabei im allgemeinen um Systeme, bei denen die imprägnierte Matrix mit Hilfe eines Kolbens oder Stempels zusammengepreßt und entspannt werden kann, wobei gleichzeitig durch einen Über- oder Unterdruck der zum Ausspülvorgang erforderliche Flüssigkeitstransport stattfindet. Dieses System kann auch mit einer als Reaktions- und/oder Meßraum dienenden Vorrichtung, z.B. einer Küvette, kombiniert werden, so daß ein Gesamtsystem erhalten wird, in dem nach Herstellen der Reaktionslösung sofort durch Zugabe der zu untersuchenden Lösung die Bestimmungsreaktion in der Küvette durchgeführt werden kann.

Das Imprägnieren der Matrix erfolgt in der Regel durch Tränken mit Lösungen, die die entsprechenden Substanzen enthalten und anschließendes Entfernen des Lösungsmittels. Als Reagenzlösungsmittel können alle geeigneten wäßrigen oder nichtwäßrigen Lösungsmittel eingesetzt werden, vorzugsweise wäßrige Lösungen wie Wasser oder Pufferlösungen.

Das Entfernen des Lösungsmittels kann durch Einfrieren der aufgegebenen Lösungen und anschließende Lyophilisation erfolgen. Dieses Verfahren ist jedoch relativ energie- und zeitaufwendig. Überraschenderweise wurde festgestellt, daß bei dem erfindungsgemäßen Verfahren auch bei größeren Wasservolumina ein Trocknen im Unterdruck bei Temperaturen oberhalb von 0°C ohne Substanzverlust möglich ist. Dies gestattet es, Substanzen mit einer geringen Löslichkeit oder in größerer Menge auf einen Träger aufzubringen, ohne daß das aufwendigere Verfahren der Lyophilisation angewendet werden muß. So ist es auch im Gegensatz zu den bekannten Verfahren möglich, Puffersubstanzen mit auf die Matrix aufzugeben, so daß für die Rekonstitution der Reagenzlösung für verschiedene Testlösungen das gleiche Reagenzlösungsmittel Verwendung finden kann. Darüberhinaus zeigt es sich, daß die bei Temperaturen über 0°C getrockneten Substanzen zum Teil geringere Trocknungsverluste aufweisen als die durch Lyophilisation erhaltenen. Dies gilt insbesondere für Proteine, was aufgrund der Temperaturempfindlichkeit dieser Substanzen als besonders überraschend angesehen werden muß.

Die Imprägnierung erfolgt unter für die einzelnen Substanzen optimierten Bedingungen. So werden unverträgliche Substanzen in getrennten Bereichen der Matrix aufgegeben, ebenso können Zusätze und pH-Werte der einzelnen aufzugebenden Lösungen verschieden sein. Insgesamt wird durch das erfindungsgemäße Verfahren gewährleistet, daß die bei der Elution erhaltene Gesamtlösung die für ein analytisches Testsystem erforderlichen Bedingungen erfüllt. Damit wird sowohl der Stabilität der einzelnen Testsubstanzen als auch den Reaktionsbedingungen des gesamten Testsystems Rechnung getragen.

Die folgenden Beispiele dienen der Erläuterung der Erfindung. Dabei finden folgende verschiedene Matrices Verwendung:

1. Ein Baumwollgewebe als Vergleichstextilgewebe mit einem Flächengewicht von 120 g/m² im folgenden "Baumwolle" genannt,

2. als Vergleichspapierträger ein Verbandszellstoff, A DIN 19310 Flächengewicht 124 g/m², im folgenden "Papier" genannt sowie

3. als Matrix für des erfindungsgemäße Verfahren ein Polyurethan-Polyesterschaumstoff, ME 6060 der Firma Metzeler, Memmingen, im folgenden "Schaumstoff" genannt.

## Beispiel 1

Herstellung einer NADH-enthaltenden Reagenzlösung

Auf je 6 Scheiben (Ø 5 12 mm) einer Matrix aus Baumwolle, Papier und Schaumstoff (Höhe 8 mm) wurden zwischen 20 und 70 µl einer 23 mmol/l NADH enthaltenden Lösung von 0,03 mol/l Bis-Tris-Puffer, pH 8,5 und 50 mmol/l EDTA aufgegeben und im Vakuum bei Zimmertemperatur getrocknet. Anschließend wurden die einzelnen Scheiben jeweils in eine Einwegspritze (Ø 5 12 mm) eingesetzt. Durch Auf- und Absenken des Spritzenkolbens wurden dann 1 ml einer Lösung von 0,05 mol/l Phosphatpuffer, pH 7,0 durch die Matrix in die Spritze aufgesaugt und wieder in das Vorratsgefäß ausgedrückt. Dieser Vorgang wurde zweimal wiederholt. In der erhaltenen Reagenzlösung wurde nach dem ersten und dem dritten Spülvorgang der NADH-Gehalt der Lösung spektrophotometrisch als Mittelwert der 6 Parallelversuche ermittelt und in Relation zur eingesetzten Menge gesetzt. Dabei ergaben sich folgende Resultate:

| aufgegebenes Reagenz-volumen | 20 µl | 20 µl | 50 µl | 50 µl | 70 µl | 70 µl |
|---|---|---|---|---|---|---|
| Anzahl der Spülvorgänge | 1 | 3 | 1 | 3 | 1 | 3 |
| % Wieder-findung bei Baumwolle | 21 | 81 | 30 | 82 | 25 | 83 |
| Papier | 68 | 95 | 67 | 70 | 62 | 95 |
| Schaumstoff | 92 | 100 | 85 | 100 | 80 | 98 |

Der Schaumstoff zeigt sich als Matrix deutlich überlegen; schon bei einem Spülvorgang werden zufriedenstellende Resultate erhalten, mehrfaches Spülen ermöglicht eine vollständige Wiederfindung.

## Beispiel 2

Herstellung einer Laktat-Dehydrogenase enthaltenden Reagenzlösung

Analog zu Beispiel 1 wurden jeweils 6 Scheiben (Ø 5 12 mm) der drei verschiedenen Matrices mit verschiedenen Volumina einer Lösung von 40 kU/l LDH in 0,1 mol/l Phosphatpuffer, pH 7,0 getränkt und bei Zimmertemperatur im Vakuum getrocknet. Die Herstellung der Reagenzlösung erfolgte durch dreimaligen Spülvorgang der in Einwegspritzen eingelegten Matrixscheiben mit jeweils 1 ml einer Lösung von 0,05 mol/l Phosphatpuffer, pH 7,0. Dabei wurden als Mittelwerte der 6 Parallelversuche folgende Ergebnisse erhalten:

| aufgegebenes Reagenzvolumen | 20 µl | 50 µl | 70 µl |
|---|---|---|---|
| % Wiederfindung bei | | | |
| Baumwolle | 2 | 7 | 6 |
| Papier | 1 | 1 | 1 |
| Schaumstoff | 96 | 82 | 94 |

Die Tabelle zeigt, daß nur der Schaumstoff als Trägermatrix geeignete Eigenschafte für das erfindungsgemäße Verfahren besitzt.

Beispiel 3

Herstellung verschiedener Reagenzlösungen

Je 6 Scheiben (Ø 5 12 mm) der drei verschiedenen Matrices wurden mit den folgenden Lösungen getränkt und anschließend im Vakuum bei Zimmertemperatur getrocknet:

G—6—PDH:      20 µl einer Lösung von 75 kU/l Glucose-6-phosphatdehydrogenase in 0,05 mol/l Triethanolamin, pH 7,5

GIDH:         25 µl einer Lösung von 4000 kU/l Glutamatdehydrogenase in 0,05 mol/l Bis-Tris-Puffer, pH 7,6, 50 mmol/l EDTA und 10 mmol/l ADP

Ketoglutarat:  15 µl einer Lösung von 1,8 mol/l Ketoglutarat in 0,03 mol/l Phosphatpuffer, pH 7,0

Die Herstellung der Reagenzlösungen erfolgt durch dreimaligen Durchspülvorgang der erhaltenen Matrixlagen mit 1 ml einer 0,05 mol/l Phosphatpuffer, pH 7,0, enthaltenden Lösung nach dem in Beispiel 1 beschriebenen Verfahren. Der Endgehalt der jeweiligen Testsubstanz wurde spektrophotometrisch bestimmt und in Relation zur Ausgangslösung gesetzt. Dabei ergaben sich folgende Werte:

| Reagenz | % Wiederfindung bei | | |
|---|---|---|---|
| | Baumwolle | Papier | Schaumstoff |
| G-6-PDH | 16 | 16 | 100 |
| G lDH | 21 | 62 | 95 |
| Ketoglutarat | 82 | 86 | 93 |

Auch in diesem Falle zeigt der Schaumstoff im erfindungsgemäßen Verfahren überlegene Eigenschaften.

Beispiel 4

Herstellung einer Gesamtreaktionslösung zur Bestimmung von Glucose

Scheiben aus Schaumstoff (Ø 5 18 mm, Höhe 8 mm) wurden mit folgenden Lösungen getränkt und bei Zimmertemperatur im Vakuum getrocknet:

4 Scheiben mit 0,5 ml einer Lösung enthaltend 0,7 mol/l Phosphatpuffer, pH 7,6 und 10 mmol/l EDTA,

1 Scheibe mit 0,5 ml einer Lösung enthaltend 1000 kU/l Glucosedehydrogenase und 22 kU/l Mutarotase in 0,1 mol/l Phosphatpuffer, pH 7,6 und 50 mmol/l EDTA,

1 Scheibe mit 0,5 ml einer Lösung von 200 mmol/l NAD in 0,1 mol/l Phosphatpuffer, pH 7,6 und 50 mmol/l EDTA.

Die getrockneten Scheiben, die in trockener Atmosphäre ein Jahr lang bei Raumtemperatur stabil sind, wurden in eine Vorrichtung entsprechend Abbildung 2 eingelegt. Zur Herstellung der Reaktionslösung zur Bestimmung von Glucose wurde diese Vorrichtung in ein Vorratsgefäß enthaltend 100 ml destilliertes Wasser gehalten und fünfmal zügig die Schaumstoffscheiben zusammengepreßt und wieder entspannt, wobei jeweils das im Vorratsgefäß befindliche Wasser in die Matrix ein- und wieder ausströmen konnte. Nach diesem Vorgang standen 100 ml einer Reaktionslösung mit folgender Zusammensetzung zur Verfügung:

0,034 mmol/l Phosphatpuffer, pH 7,6

5,00 kU/l Glucosedehydrogenase

0,11 kU/l Mutarotase

1,00 mmol/l NAD

Der Glucosegehalt in der Untersuchungslösung wurde ermittelt, indem zu 2 ml dieser Reaktionslösung 20 µl Probe hinzugegeben wurden. Nach 10 Min. Inkubation bei Zimmertemperatur wurde die Extinktion bei 334 nm gemessen. Die Konzentration der Glucose ergibt sich dabei nach der Gleichung:

$$c \; [mg/100 \; ml] = 294 \cdot E$$

**Beispiel 5**

Herstellung einer Gesamtreaktionslösung zur Bestimmung von Glutamat-Oxalacetat-Transaminase (GOT)

Folgende Lösungen wurden auf Schaumstoffscheiben (Ø 5 18 mm) aufgegeben: 0,5 ml 1,6 mol/l Phosphatpuffer, pH 7,6 auf eine Scheibe (Höhe 8 mm),

1,4 ml einer Lösung von 1,5 mol/l Aspartat in 0,1 mol/l Phosphatpuffer, pH 7,0 auf eine Scheibe (Höhe 22 mm),

0,8 ml einer Lösung von 180 mmol/l Ketoglutarat in 0,1 mol/l Phosphatpuffer, pH 7,0 und 50 mmol/l EDTA auf eine Scheibe (Höhe 16 mm),

0,01 ml einer Lösung von 18 mmol/l NADH in 0,05 mol/l Bis-Trispuffer, pH 8,5 und 50 mmol/l EDTA auf eine Scheibe (Höhe 4 mm) und

0,02 ml einer Lösung von 60 kU/l LDH und 30 kU/l MDH in 0,1 mol/l Phosphatpuffer, pH 7,0 auf eine Scheibe (Höhe 4 mm).

Nach dem Trocknen wurden die Scheiben nebeneinander in eine Vorrichtung entsprechend Abbildung 1 gegeben und nach dem beschriebenen Verfahren mit 10 ml dest. Wasser fünfmal ausgespült. Es wurden 10 ml einer Reaktionslösung folgender Zusammensetzung erhalten:

0,08 Mol/l Phosphatpuffer, pH 7,0

200,00 mmol/l Aspartat

12,00 mmol/l Ketoglutarat

0,18 mmol/l NADH

1,20 kU/l LDH und

0,60 kU/l MDH

Diese Reaktionslösung ist für die Bestimmung der GOT-Aktivität nach bekannten Methoden geeignet.

**Patentansprüche**

1. Verfahren zur Herstellung von mehrere Substanzen enthaltenden Reagenzlösungen zur Durchführung von Analysen, wobei die Substanzen auf mindestens ein Element eines festen porigen und preßfähigen Trägermaterials aufgebracht sind, dadurch gekennzeichnet, daß die Herstellung der Reagenzlösung durch Einsaugen eines Reagenzlösungsmittels in das Trägermaterial und anschließendes mehrfaches Ausdrücken und Entspannen in dem Reagenzlösungsmittel erfolgt.

2. Verfahren nach Anspruch 1, dadurch gekennzeichnet, daß als poriges und preßfähiges Trägermaterial ein offenzelliger Polyurethanschaumstoff verwendet wird.

3. Verfahren nach den Ansprüchen 1 und 2, dadurch gekennzeichnet, daß als Reagenzlösungsmittel wäßrige Lösungen verwendet werden.

6

# EP 0 192 129 B1

**Revendications**

1. Procédé de préparation de solutions de réactifs contenant plusieurs substances pour l'exécution d'analyses, dans lequel les substances sont appliquées sur au moins un élément d'une matière de support solide poreuse et compressible, caractérisé en ce que la solution de réactif est préparée par aspiration d'un solvant du réactif dans la matière de support suivie de compressions et détentes répétées dans le solvant du réactif.

2. Procédé selon la revendication 1, caractérisé en ce que l'on utilise en tant que matière de support poreuse et compressible une mousse de polyuréthanne à cellules ouvertes.

3. Procédé selon les revendications 1 et 2, caractérisé en ce que l'on utilise en tant que solvants des réactifs, des solutions aqueuses.

**Claims**

1. Method for the preparation of reagent solutions containing several substances for carrying out analyses, the substances being applied to at least one element of a solid porous and compressible carrier material, characterized in that the preparation of the reagent solution is carried out by aspiration of a reagent solvent into the carrier material followed by squeezing out and relaxing many times in the reagent solvent.

2. Method according to Claim 1, characterized in that the porous and compressible carrier material used is an open-cell polyurethane foam.

3. Method according to Claims 1 and 2, characterized in that the reagent solvents used are aqueous solutions.

Abb. 1

Abb. 2